# FASCICULE DE BREVET EUROPEEN

(11) **EP 1 753 353 B1**
(45) Date de publication et mention de la délivrance du brevet: **09.11.2011**
(21) Numéro de dépôt: 05773210.9
(22) Date de dépôt: 26.05.2005
(51) Int. Cl.: A61B 17/70, A61F 2/44

(54) **SYSTEME D'ARTHROPLASTIE RACHIDIENNE**
BANDSCHEIBENIMPLANTATSYSTEM
SPINAL ARTHOPLASTY SYSTEM

(30) Priorité: 27.05.2004 FR 0405720
(43) Date de publication de la demande: 21.02.2007
(73) Titulaire: ZIMMER SPINE, 33000 Bordeaux (FR)
(72) Inventeur: LE COUEDIC, Régis, F-78570 Andresy (FR)
(74) Mandataire: Besnard, Christophe Laurent
(86) Numéro de dépôt international: PCT/FR2005/001301
(87) Numéro de publication internationale: WO 2005/117728

(56) Documents cités:
- EP-A- 0 747 025
- WO-A1-02/071960
- WO-A2-03/015646
- WO-A2-03/045262
- US-A- 5 425 773
- US-B1- 6 572 653

## Description

La présente invention a pour objet un système d'arthroplastie rachidienne.

Le principe du remplacement d'un disque intervertébral par une prothèse de type mécanique afin de réaliser une arthroplastie rachidienne est connu et décrit dans de nombreux documents.

Cette opération visant à traiter les pathologies dégénératives du disque intervertébral peut être réalisée par un abord chirurgical antérieur ou par un abord postérieur.

Une autre technique pour permettre le traitement de ce type de pathologie consiste à utiliser des implants permettant le remplacement du seul nucléus pulposus du disque intervertébral. Ces implants sont dits prothèses de nucléus. La prothèse est insérée dans la partie centrale du disque intervertébral après ablation du nucléus. Ils sont en général constitués d'un matériau hydrophile s'expansant jusqu'à sa taille définitive dans les 24 ou 48 heures qui suivent l'opération et présentent des caractéristiques viscoélastiques tendant à reproduire le comportement mécanique du nucléus.

Toutefois, les indications relatives à l'utilisation des prothèses de disque intervertébral, quelque soit la voie d'abord utilisée ou l'utilisation de prothèse de nucléus, sont limitées par l'état des structures postérieures du niveau vertébral à traiter. On entend par structure postérieure, les articulations postérieures réalisées entre les facettes articulaires des vertèbres disposées au-dessus et en dessous du disque malade. Dans les cas où les articulations postérieures sont elles-mêmes atteintes par la dégénérescence, l'indication de prothèse de disque intervertébral ou de prothèse de nucléus est alors souvent jugée trop sévère pour qu'une prothèse discale partielle ou totale puisse être utilisée dans de bonnes conditions et avec des chances réelles de succès. Cela est dû notamment au fait qu'aussi bien dans le cas de prothèse intervertébrale mécanique que de prothèse de nucléus, ces dispositifs visant à remplacer au moins partiellement le disque intervertébral naturel ne comportent pas de butée mécanique des mouvements relatifs des vertèbres adjacentes. Dans ce cas, les vertèbres elles-mêmes et du fait de leur dégénérescence ne constituent plus ces butées mécaniques de limitation de mouvement relatif. Le chirurgien n'a donc souvent pas d'autres alternatives que de procéder à une fusion du niveau vertébral concerné en utilisant par exemple un système comportant des vis pédiculaires reliées à des tiges pour immobiliser les deux vertèbres, ce qui bien sûr peut diminuer le confort du patient.

Un autre exemple de système d'arthroplastie rachidienne est divulgué dans la demande PCT m° WO 03/015646 A2.

Un objet de la présente invention est de fournir un système d'arthroplastie rachidienne qui permet l'utilisation de prothèses de disque intervertébral totales même dans le cas de dégénérescence sévère des éléments articulaires postérieurs des vertèbres disposées au-dessus et en dessous du disque malade.

Pour atteindre ce but selon l'invention, le système d'arthroplastie rachidienne destiné à être mis en place entre deux vertèbres contiguës se caractérise en ce qu'il comprend une prothèse totale de disque intervertébral choisie dans le groupe des prothèses mécaniques rotulantes et une cale destinée à être disposée entre les apophyses épineuses des deux vertèbres, ladite cale comportant à chacune de ses extrémités un logement pour recevoir une des apophyses épineuses, et au moins un lien pour maintenir chaque apophyse dans un logement de ladite cale.

On comprend que grâce à l'utilisation de l'implant intervertébral disposé entre les apophyses épineuses des vertèbres disposées de part et d'autre du disque malade, on met en oeuvre la fonction de butée mécanique de mouvement relatif que les vertèbres objet de la dégénérescence ne pouvaient plus mettre en oeuvre. Ainsi, il est possible d'utiliser un implant mécanique rotulant de disque intervertébral ou un implant de nucléus dans des conditions optimales. En effet, la cale de l'implant intervertébral permet de limiter les mouvements relatifs des deux vertèbres dans les différents plans de mobilité.

Selon un premier mode de mise en oeuvre, le système comprend une prothèse de disque intervertébral comportant un premier ensemble présentant une face de fixation sur une vertèbre et une face active dont une partie a la forme d'une première calotte sensiblement sphérique concave, et un deuxième ensemble présentant une face de fixation sur l'autre vertèbre et une face active dont une partie a la forme d'une deuxième calotte sensiblement sphérique convexe pour coopérer avec la première calotte sphérique. Comme cela est par ailleurs bien connu, la prothèse de disque constituée par les deux éléments comportant les calottes sphériques conjuguées permet d'autoriser des mouvements relatifs des deux vertèbres proches des deux mouvements relatifs naturels.

D'autres caractéristiques et avantages de l'invention apparaîtront mieux à la lecture de la description qui suit de plusieurs modes de réalisation de l'invention donnés à titre d'exemples non limitatifs. La description se réfère aux dessins annexés sur lesquels :
- la figure 1 est une vue simplifiée du premier mode de mise en oeuvre avec une prothèse de disque, intervertébral ;
- la figure 2 est une vue simplifiée d'un système utilisant une prothèse de nucléus, me faisant pas partie de l'invention définie dans la revendication 1 annexée ;
- les figures 3A et 3B illustrent en coupe diamétrale un premier mode de réalisation de la prothèse de disque intervertébral ;
- la figure 4 montre en perspective un deuxième mode de réalisation de la prothèse de disque intervertébral ;
- les figures 5A, 5B et 5C montrent les différentes parties de la prothèse de disque intervertébral représentée sur la figure 4 ;
- la figure 6 montre en perspective un troisième mode de réalisation de la prothèse de disque intervertébral ;
- les figures 7A à 7E montrent en détail les différentes parties de la prothèse de disque intervertébral montrée sur les figures 6 ;
- la figure 8 montre en perspective un premier mode de réalisation de l'implant intervertébral ; et
- la figure 9 montre en perspective un deuxième mode de réalisation de l'implant intervertébral.

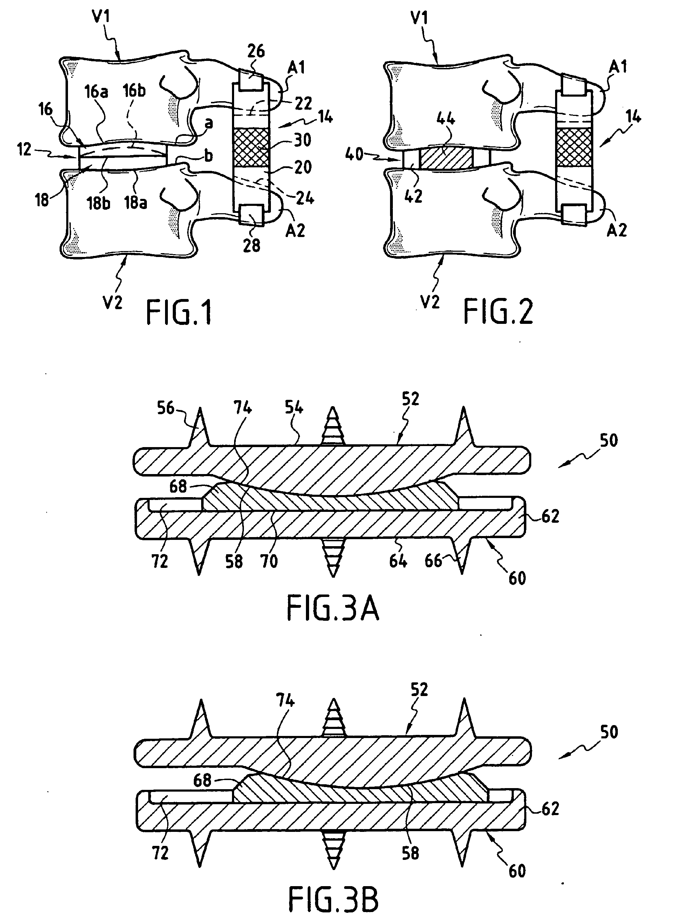

Sur la figure 1, on a représenté de façon simplifiée un premier mode de réalisation du système d'arthroplastie rachidienne selon l'invention.

Sur cette figure, apparaissent les vertèbres V1 et V2 ainsi que leurs apophyses épineuses A1 et A2. Dans ce mode de réalisation, le système d'arthroplastie rachidienne est constitué par une prothèse de disque intervertébral 12 et par un implant intervertébral 14. La prothèse de disque intervertébral 12 est montée entre les plateaux a et b des vertèbres V1 et V2. La prothèse 12 est essentiellement constituée par deux éléments 16 et 18 qui présentent une face 18a, 16a de fixation sur les plateaux des vertèbres et une face 16b, 18b définissant une face commune de contact en forme de calotte sphérique. Grâce aux surfaces de contact 18b et 16b, les deux éléments de la prothèse peuvent se déplacer de façon relative autorisant ainsi au moins une partie du mouvement naturel relatif des vertèbres V1 et V2.

L'implant intervertébral 14 est essentiellement constitué par une cale 20 qui présente à ses extrémités des gorges 22 et 24 destinées à recevoir les apophyses épineuses A1 et A2. La solidarisation de la cale 14 avec les épineuses A1 et A2 est réalisée par deux sangles 26 et 28 dont les extrémités sont solidaires de la cale 20 et qui entourent les apophyses. De préférence, la partie centrale 30 de la cale 14 est élastiquement déformable, ce qui autorise un certain mouvement relatif entre les vertèbres V1 et V2.

On comprend que dans le cas considéré pour l'invention, c'est-à-dire dans le cas où les articulations postérieures des vertèbres sont atteintes par une dégénérescence, l'implant intervertébral 14 joue le rôle de limitateur des mouvements relatifs des deux vertèbres qu'auraient joué normalement les articulations postérieures lorsqu'elles sont en bon état. On comprend également que la combinaison de la prothèse de disque intervertébral et de l'implant intervertébral permet de résoudre le problème du remplacement du disque intervertébral naturel dans les conditions particulières considérées.

Sur la figure 2, on a représenté également de façon simplifiée un système d'arthroplastie rachidienne ne faisant par partie de l'invention. Le disque intervertébral naturel 40 présente une dégénérescence plus limitée. Dans ce cas, au lieu d'utiliser la prothèse 12, on conserve la partie périphérique 42 du disque naturel et on remplace son nucléus pulposus à l'aide d'une prothèse de nucléus 44 qui est substituée à la partie centrale du disque naturel. Les prothèses de nucléus sont en elles-mêmes connues ainsi qu'on l'a déjà indiqué et sont en général constituées d'un matériau hydrophile s'expansant jusqu'à sa taille définitive dans les 24 ou 48 heures suivant l'opération. Ces prothèses de nucléus qui constituent en fait une prothèse partielle du disque naturel présentent des caractéristiques viscoélastiques tendant à reproduire le comportement mécanique du nucléus. Il faut souligner que l'utilisation d'une prothèse de nucléus n'est possible que si les structures postérieures des vertèbres sont en parfait état puisque la prothèse de nucléus ne permet aucun guidage des mouvements relatifs des deux vertèbres, alors que les prothèses de disque intervertébral permettent un certain guidage limité.

Des prothèses de nucleus de disques intervertébraux sont décrites dans European Spine Journal Volume 11, Supplément 2, Octobre 2002 et dans SPINE Volume 27 (11) 1er juin 2002 pp 1245 à 1247.

Dans ce système d'arthroplastie rachidienne, la prothèse de nucléus 44 est complétée par un implant intervertébral 14 identique à celui qui a été décrit en liaison avec la figure 1. Ainsi qu'on l'a déjà expliqué l'implant intervertébral est disposé entre les apophyses épineuses A1 et A2, et sert à limiter les possibilités de mouvements relatifs entre les deux vertèbres pour se substituer dans cette fonction aux structures postérieures des vertèbres qui sont dégénérées.

On comprend que selon le premier mode de réalisation de l'invention, le système d'arthroplastie rachidienne est constitué par une prothèse de disque intervertébral qui peut présenter un grand nombre de structures différentes et par un implant intervertébral de limitation de mouvement relatif qui peut également présenter de nombreuses structures différentes.

Dans la description qui suit, on va décrire différentes prothèses de disque intervertébral utilisables dans l'invention, ainsi que différents implants intervertébraux également utilisables dans cette invention.

Sur les figures 3A et 3B on a représenté un premier exemple de réalisation d'implant de disque intervertébral qui porte la référence 50. Le premier ensemble 52 de la prothèse 50 comporte une face de fixation 54 munie de moyens d'ancrage 56 dans le plateau de la vertèbre et une face de contact 58 en forme de calotte sphérique. Le deuxième élément 60 de la prothèse est constitué par une pièce de fixation 62 dont la face de fixation 64 est munie d'organes d'ancrage tels que 66 et par une pièce de contact 68. La pièce 68 comporte une face de glissement 70 qui peut coopérer avec le fond d'un lamage 72 réalisé dans la première pièce 62. La deuxième pièce 68 comporte également une face active 74 en forme de calotte sphérique pour coopérer avec la surface en forme de calotte sphérique 58 du premier ensemble 52.

On comprend que cette prothèse de disque intervertébral permet non seulement un mouvement relatif rotulant grâce à la présence des deux calottes sphériques mais également un mouvement de translation. Une telle prothèse de disque intervertébral est décrite plus en détails dans la demande de brevet PCT WO 00/53127. D'autres prothèses d'implant intervertébral sont décrites par exemple dans la demande de brevet européen EP 176 728.

Dans tous les cas, la mise en place de la prothèse d'implant intervertébral du type décrit précédemment se fait par abord antérieur. On comprend que le plus souvent une telle solution n'est pas optimale puisqu'en revanche l'implant intervertébral 14 doit être mis en place par abord postérieur.

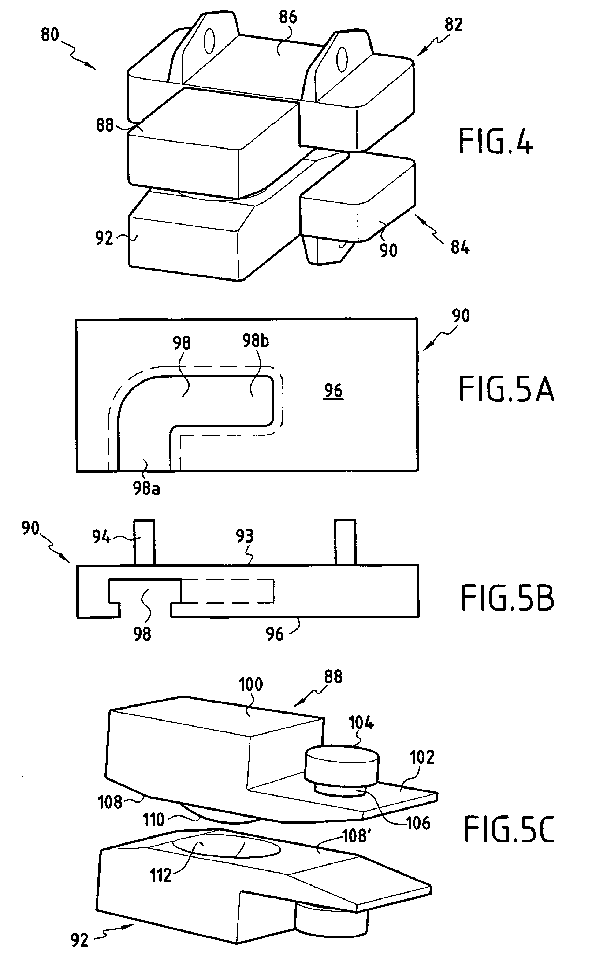

Pour remédier à cet inconvénient, il est souhaitable d'utiliser une prothèse d'implant de disque intervertébral susceptible d'être mise en place par abord postérieur. On va décrire deux exemples préférés de prothèse de ce type par référence aux figures 4 et 5 d'une part et 6 et 7 d'autre part.

En se référant tout d'abord aux figures 4 et 5A à 5C, on va décrire une prothèse de disque intervertébral 80 qui peut être mise en place par abord postérieur. La prothèse 80 comporte un premier ensemble de prothèse 82 et un deuxième ensemble de prothèse 84. Le premier ensemble 82 est constitué par un premier élément de fixation 86 et par un premier élément de prothèse 88 alors que le deuxième ensemble 84 est constitué par un deuxième élément de fixation 90 et par un deuxième élément de prothèse 92.

En se référant aux figures 5A et 5B, on va décrire l'élément de fixation 90, l'élément de fixation 86 lui étant identique. L'élément 90 a la forme générale d'une plaque rectangulaire comportant une face de fixation 93 munie de moyens d'ancrage 94 dans le plateau de la vertèbre et une face opposée de coopération 96.

Dans la face de coopération 96 est ménagée une rainure 98 débouchant dans la face 96 et dont la section droite est en forme de T comme le montre la figure 5B. La rainure 98 présente une portion d'engagement 98a qui débouche dans un des rebords de la pièce 90 et par une deuxième partie 98b qui lui est perpendiculaire.

La figure 5C montre les éléments de prothèse 88 et 92. L'élément de prothèse 88 comporte une face de coopération 100 qui comporte une portion en retrait 102 dans laquelle est réalisé un organe de verrouillage 104 constitué par un téton dont la partie de raccordement 106 à la surface 102 a un diamètre réduit. Les dimensions du téton 104 sont déterminées de telle manière qu'il puisse être engagé dans la rainure 98. L'élément de prothèse 88 comporte également une face active 108 qui définit essentiellement une surface en forme de calotte sphérique convexe 110.

L'élément de prothèse 92 présente la même architecture que l'élément de prothèse 88 à l'exception du fait que sa face active 108' définit une surface en forme de calotte sphérique concave 112 apte à coopérer avec la calotte sphérique convexe 110 de l'élément de prothèse 88.

La mise en place par abord postérieur de la prothèse de disque intervertébral 80 est réalisée de la manière suivante :
dans une première phase le chirurgien met en place successivement ou simultanément les éléments de fixation 82 et 84. Cette mise en place par abord postérieur est possible du fait de la dimension relativement réduite des éléments de fixation. Dans un deuxième temps, le chirurgien introduit successivement chaque élément de prothèse 88 et 92 de telle manière que le téton d'ancrage 104 pénètre dans la partie d'introduction de la rainure 98 de l'élément de fixation correspondant. Puis par un pivotement de 90° le chirurgien déplace le téton 104 dans la deuxième partie 98b de la rainure 98 jusqu'à obtention de son immobilisation. On obtient ainsi un verrouillage de chaque élément de prothèse sur l'élément de fixation. Il faut souligner que grâce aux modes de réalisation des éléments de verrouillage respectifs (rainure 98, téton 104) le verrouillage des éléments de prothèse sur les éléments de fixation se fait uniquement par un mouvement des éléments de prothèse dans un plan perpendiculaire à l'axe commun des deux vertèbres entre lesquelles la prothèse doit être mise en place.

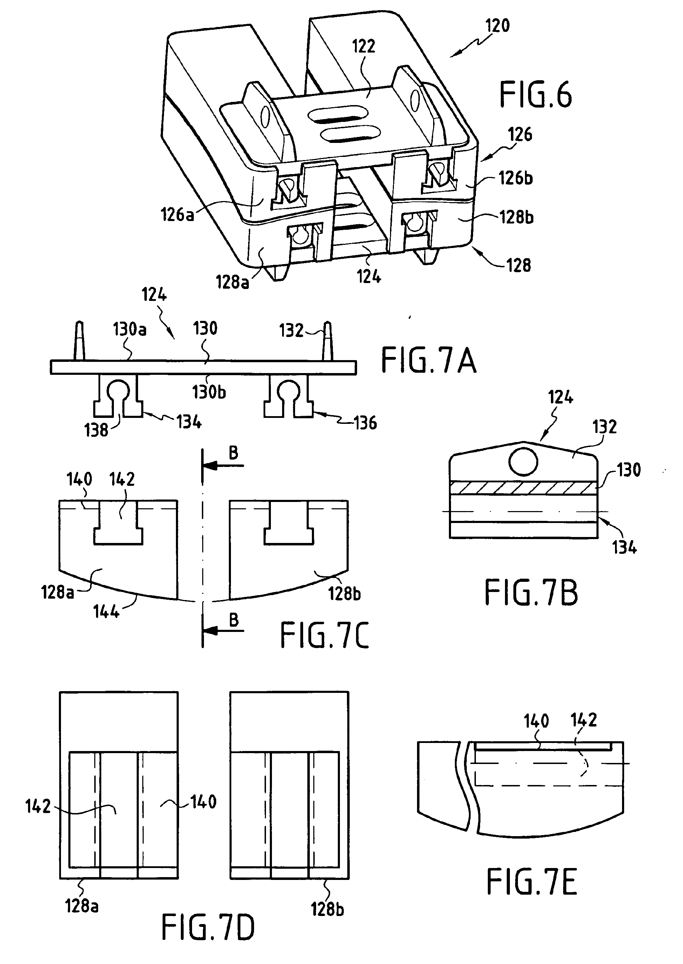

En se référant maintenant aux figures 6 et 7A à 7E, on va décrire un deuxième exemple de prothèse de disque intervertébral qui peut être mis en place par abord postérieur.

La figure 6 montre que la prothèse 120 est constituée par deux éléments de fixation 122 et 124 et par deux éléments de prothèse 126 et 128 chaque élément de prothèse étant constitué par deux pièces distinctes 126a et 126b pour l'élément 126 et 128a et 128b pour l'élément 128.

Les figures 7A et 7B montrent l'élément de fixation 124, l'élément de fixation 122 lui étant identique. L'élément de fixation 124 est constitué par une plaque sensiblement rectangulaire 130 dont la face de fixation 130a est équipée d'organes d'ancrage tels que 132. L'autre face 130b de la plaque 130 est équipée de deux organes de verrouillage 134 et 136. Chaque organe de verrouillage 134, 136 a la forme d'une barrette dont la section droite est en forme de T. De préférence, chaque organe de verrouillage comporte une rainure longitudinale 138 pour conférer aux organes de verrouillage une certaine élasticité.

Les figures 7C à 7E montrent les pièces 128a et 128b de l'élément de prothèse 128.

Chaque pièce 128a et 128b de l'élément de prothèse 128 comporte dans sa face de coopération 140 une rainure débouchante 142 en forme de T apte à recevoir les organes de verrouillage 134 et 136. On comprend que lorsque les pièces 128a et 128b sont solidarisées avec la plaque 130, ces pièces sont immobilisées l'une par rapport à l'autre. Les faces de contact 144 des pièces 128a et 128b définissent une portion de calotte sphérique. Les deux portions de calottes sphériques ainsi définies appartiennent à une même calotte sphérique lorsque les pièces 128a et 128b sont solidarisées l'une à l'autre à l'aide de la pièce de l'élément de fixation 124. De la même manière, les pièces 126a et 126b ont une face active dont la réunion définit une calotte sphérique concave.

On comprend que le mode de réalisation de la prothèse de disque intervertébral qui vient d'être décrit est particulièrement bien adapté à sa mise en place par abord postérieur. En effet, le chirurgien peut dans un premier temps mettre en place sur les plateaux vertébraux simultanément ou successivement les deux éléments de fixation 122 et 124. Ensuite, il met en place de part et d'autre de la moelle épinière les pièces 126a et 126b constituant l'élément de prothèse 126 en verrouillant celles-ci sur l'élément de fixation 122 puis, de la même manière, il met en place les pièces 128a et 128b de l'élément de prothèse 128 et verrouille celles-ci sur l'élément de fixation 124. De plus, comme dans le mode de réalisation décrit précédemment, la mise en place et le verrouillage des pièces constituant les éléments de prothèse peut être réalisée par des déplacements de ces pièces dans un plan sensiblement perpendiculaire à l'axe commun des vertèbres entre lesquelles la prothèse doit être placée.

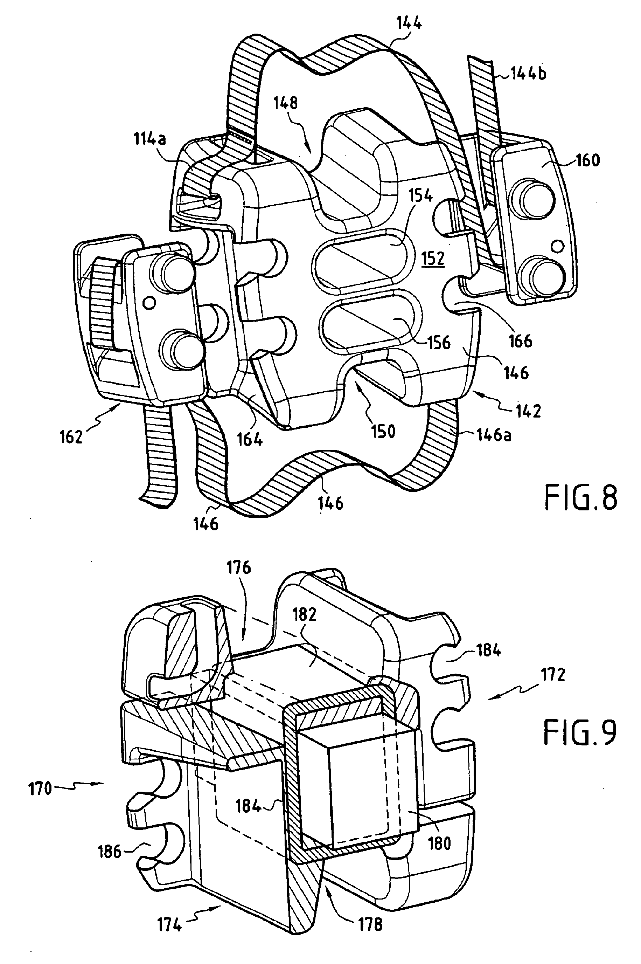

En se référant maintenant aux figures 8 et 9, on va décrire deux exemples de réalisation d'implants intervertébraux utilisables dans le système d'arthroplastie rachidienne.

Sur la figure 8, l'implant 140 est constitué ainsi qu'on l'a déjà expliqué par une cale interépineuse 142 et deux sangles ou ligaments 144 et 146 pour solidariser les extrémités de la cale avec les apophyses épineuses des vertèbres. La cale 142 comprend un corps dont les extrémités comportent des gorges ou logements 148 et 150 destinées à recevoir les apophyses épineuses. La partie centrale 152 du corps de la cale comporte des évidements 154, 156 parallèles aux gorges 148 et 150. Les évidements 154 et 156 ont pour but de conférer une certaine élasticité ou flexibilité au corps de la cale et donc d'autoriser un certain mouvement relatif entre les deux vertèbres. Les sangles 144 et 146 présentent une première extrémité 146a, 144a qui est solidaire du corps de la cale et une deuxième extrémité 144b, 146b qui est engagée dans des dispositifs de verrouillage autobloquant 160 et 162 qui peuvent être clipsés sur les parois latérales 164 et 166 du corps de cale. Cet implant intervertébral est décrit plus en détails dans la demande de brevet WO 02/071960 au nom de la demanderesse.

L'implant intervertébral représenté sur la figure 9 est décrit en détails dans la demande de brevet français 01 015 494 au nom de la demanderesse.

La cale 170 représentée sur la figure 9 est constituée par deux pièces séparées respectivement référencées 172 et 174. La pièce 172 définit une gorge supérieure 176 pour une apophyse épineuse et la pièce 174 définit une gorge inférieure 178 pour l'apophyse épineuse de l'autre vertèbre. Les parois internes des pièces 172 et 174 définissent un volume V sensiblement parallélépipédique. A l'intérieur de ce volume V est montée une pièce également sensiblement parallélépipédique 180 réalisée en un matériau élastomérique présentant des propriétés de compressibilité. Les deux pièces 172 et 174 sont maintenues entre elles par un lien 182 engagé dans des passages réalisés dans les pièces 172 et 174 de telle manière que le lien 182 entoure complètement le volume V. En outre, chaque pièce 172 et 174 de la cale 170 est équipée de moyens de clipsage 184 et 186 pour la fixation de systèmes autobloquants analogues aux systèmes 160 et 162 de la figure 8. Ces systèmes permettent de fixer avec serrage des liens analogues aux liens 144 et 146 de la figure 8 pour assurer la solidarisation de la cale 170 avec les apophyses épineuses des vertèbres.

On comprend que grâce à la présence du bloc élastiquement déformable 180, la cale 170 autorise un mouvement de rapprochement des épineuses par compression de la pièce 180. En revanche, le lien 182 en limitant les possibilités de déplacements relatifs des pièces 172 et 174 limite les possibilités d'écartement de ces mêmes épineuses.

L'utilisation d'une cale interépinale présentant une certaine élasticité est particulièrement intéressante puisque la prothèse au moins partielle de disque intervertébral, qu'il s'agisse d'une prothèse mécanique rotulante ou d'une prothèse de nucleus de disque autorise un mouvement rotulant des deux vertèbres qui est certes limité par la cale, mais qui reste en partie autorisé.

Il va de soi que d'autres types d'implants intervertébraux disposés entre les apophyses épineuses des vertèbres entourant le disque intervertébral à remplacer pourraient être utilisés dès lors que, de préférence, il présentent certaines possibilités illimitées de déplacements relatifs des apophyses épineuses des vertèbres.

Plus généralement, de préférence, dans le mode de réalisation de la figure 8, les évidements 154 et 156 traversent la partie médiane de la cale de part en part et dans le mode de réalisation de la figure 9, la partie médiane de la cale présente un coefficient d'élasticité supérieur à celui de ses extrémités.

## Revendications

1. Système d'arthroplastie rachidienne destiné à être mis en place entre deux vertèbres contiguës, ce système comprenant un implant intervertébral (14) comportant :
- une cale (20) destinée à être disposée entre les apophyses épineuses des deux vertèbres, ladite cale (20) comportant à chacune de ses extrémités un logement (22, 24) pour recevoir une des apophyses épineuses, et
- au moins un lien (26, 28) pour maintenir chaque apophyse dans un logement (22, 24) de ladite cale (20),
ce système étant **caractérisé en ce qu'**il comprend, en outre, une prothèse totale de disque intervertébral (12) choisie dans le groupe des prothèses mécaniques rotulantes.

2. Système d'arthroplastie rachidienne selon la revendication 1 **caractérisé en ce qu'**il comprend une prothèse mécanique rotulante de disque intervertébral comportant :
- un premier ensemble (52, 82) présentant une face de fixation sur une vertèbre et une face active dont une partie a la forme d'une première calotte sensiblement sphérique convexe ; et
- un deuxième ensemble (60, 84) présentant une face de fixation sur une vertèbre et une face active dont une partie a la forme d'une deuxième calotte sensiblement sphérique concave pour coopérer avec la première calotte sphérique.

3. Système d'arthroplastie rachidienne selon la revendication 2, **caractérisé en ce que** chacun desdits premier et deuxième ensembles de ladite prothèse comprend un élément de fixation (86, 90, 122, 124) comportant ladite face de fixation et un élément de prothèse (88, 92, 126, 128) comportant ladite face active, lesdits éléments étant munis de moyens mécaniques de solidarisation mutuelle.

4. Système d'arthroplastie rachidienne selon la revendication 2 ou 3, **caractérisé en ce que** chaque élément de prothèse (126, 128) comprend deux pièces distinctes (126a, 126b, 128a, 128b) destinées à être disposées côte à côte, chaque pièce comportant une face de fixation et une face active et **en ce que** chaque ensemble de prothèse comprend en outre des moyens (122, 124) de solidarisation des deux pièces, les faces actives des deux pièces solidarisées définissant une desdites calottes sphériques.

5. Système d'arthroplastie rachidienne selon la revendication 1, **caractérisée en ce que** chaque élément de prothèse est constitué par une unique pièce.

6. Système d'arthroplastie rachidienne selon l'une quelconque des revendications 1 à 5, **caractérisé en ce que** ladite cale (170) comprend deux extrémités (172, 174) comportant chacune un desdits logements (176, 178) et une partie médiane comprenant des moyens (180) déformables en compression, actifs lors du rapprochement mutuel des deux vertèbres, et des moyens de liaison (182) pour limiter l'écartement des deux extrémités de ladite cale sous l'effet d'un mouvement d'éloignement mutuel des vertèbres.

7. Système d'arthroplastie rachidienne selon l'une quelconque des revendications 1 à 5, **caractérisé en ce que** ladite cale (140) est réalisée à l'aide d'un seul matériau et sa partie médiane (152) comporte des évidements (154, 156) la traversant de part en part.

## Claims

1. A spinal arthroplasty system intended to be placed between two contiguous vertebrae, the system comprising an intervertebral implant (14) comprising:
- a spacer (20) intended to be placed between the spinous processes of the two vertebrae, said spacer (20) having a housing (22, 24) at each of its ends for receiving one of the spinous processes; and
- at least one tie (26, 28) for holding each process in a housing (22, 24) of said spacer (20),
the system being **characterized in that** it further comprises a total prosthesis of the intervertebral disk (12) selected from the group of ball-and-socket type mechanical prostheses.

2. A spinal arthroplasty system according to claim 1, **characterized in that** it comprises a ball-and-socket type mechanical prosthesis of the intervertebral disk (12) comprising:
- a first assembly (52, 82) presenting a fastening face for fastening to a vertebra and an active face having a portion in the form of a first cap being substantially spherical convex; and
- a second assembly (60, 84) presenting a fastening face for fastening to a vertebra and an active face including a portion in the form of a second cap being substantially spherical convex, for co-operating with the first spherical cap.

3. A spinal arthroplasty system according to claim 2, **characterized in that** each of said first and second assemblies of said prosthesis includes a fastener element (86, 90, 122, 124) including said fastening face and a prosthesis element (88, 92, 126, 128) including said active face, said elements being provided with mechanical means for securing them together.

4. A spinal arthroplasty system according to claim 2 or 3, **characterized in that** each prosthesis element (126, 128) comprises two distinct parts (126a, 126b, 128a, 128b) intended to be placed side by side, each part having a fastening face and an active face, and **in that** each prosthesis assembly further comprises means (122, 124) for securing the two parts together, the active faces of the two secured parts defining one of said spherical caps.

5. A spinal arthroplasty system according to claim 1, **characterized in that** each prosthesis assembly is constituted by a single part.

6. A spinal arthroplasty system according to any one of claims 1 to 5, **characterized in that** said spacer (170) comprises two ends (172, 174), each including one of said housings (176, 178), and a middle portion including means (180) deformable in compression that are active when the two vertebrae move towards each other, and tie means (182) for limiting the extent to which the ends of said spacer can move apart under the effect of mutual separation movements between the vertebrae.

7. A spinal arthroplasty system according to any one of claims 1 to 5, **characterized in that** said spacer (140) is made out of a single material and its middle portion (152) includes recesses (154, 156) passing right through it.

## Patentansprüche

1. System zur Arthroplastik der Wirbelsäule, das dazu bestimmt ist, zwischen zwei benachbarten Wirbeln eingesetzt zu werden, wobei dieses System ein Zwischenwirbelimplantat (14) umfaßt mit:
- einem Keil (20), der dazu bestimmt ist, zwischen den Dornfortsätzen der beiden Wirbel angeordnet zu werden, wobei der Keil (20) an jedem seiner Enden eine Aufnahme (22, 24) zum Aufnehmen von einem der Dornfortsätze aufweist, und
- wenigstens einem Bindeglied (26, 28), um jeden Fortsatz in einer Aufnahme (22, 24) des Keils (20) zu halten,
wobei dieses System **dadurch gekennzeichnet ist, daß** es ferner eine Bandscheiben-Vollprothese (12) umfaßt, die aus der Gruppe der mechanischen Kugelgelenkprothesen ausgewählt ist.

2. System zur Arthroplastik der Wirbelsäule nach Anspruch 1, **dadurch gekennzeichnet, daß** es eine mechanische Bandscheiben-Kugelgelenkprothese umfaßt mit:
- einer ersten Anordnung (52, 82), die eine Fläche zur Befestigung an einem Wirbel sowie eine aktive Fläche aufweist, von welcher ein Teil die Form einer ersten, im wesentlichen konvex kugeligen Kappe aufweist, und
- einer zweiten Anordnung (60, 84), die eine Fläche zur Befestigung an einem Wirbel sowie eine aktive Fläche aufweist, von welcher ein Teil die Form einer zweiten, im wesentlichen konkav kugeligen Kappe aufweist, um mit der ersten Kugelkappe zusammenzuwirken.

3. System zur Arthroplastik der Wirbelsäule nach Anspruch 2, **dadurch gekennzeichnet, daß** eine jede der ersten und der zweiten Anordnung der Prothese ein Befestigungselement (86, 90, 122, 124), welches die Befestigungsfläche aufweist, sowie ein Prothesenelement (88, 92, 126, 128), welches die aktive Fläche aufweist, umfaßt, wobei die Elemente mit mechanischen Mitteln zum gegenseitigen festen Verbinden ausgestattet sind.

4. System zur Arthroplastik der Wirbelsäule nach Anspruch 2 oder 3, **dadurch gekennzeichnet, daß** jedes Prothesenelement (126, 128) zwei getrennte Teile (126a, 126b, 128a, 128b) umfaßt, die dazu bestimmt sind, Seite an Seite angeordnet zu werden, wobei jedes Teil eine Befestigungsfläche und eine aktive Fläche umfaßt, und daß jede Prothesenanordnung ferner Mittel (122, 124) zum festen Verbinden der beiden Teile umfaßt, wobei die aktiven Flächen der beiden fest verbundenen Teile eine der Kugelkappen definieren.

5. System zur Arthroplastik der Wirbelsäule nach Anspruch 1, **dadurch gekennzeichnet, daß** jedes Prothesenelement von einem einzigen Teil gebildet ist.

6. System zur Arthroplastik der Wirbelsäule nach einem der Ansprüche 1 bis 5, **dadurch gekennzeichnet, daß** der Keil (170) zwei Enden (172, 174), die jeweils eine der Aufnahmen (176, 178) umfassen, sowie einen Mittelteil umfaßt, der druckverformbare Mittel (180), welche bei gegenseitiger Annäherung der beiden Wirbel aktiv sind, sowie Verbindungsmittel (182) zum Begrenzen des Abstands der beiden Enden des Keils unter der Wirkung einer Bewegung zum gegenseitigen Entfernen der Wirbel umfaßt.

7. System zur Arthroplastik der Wirbelsäule nach einem der Ansprüche 1 bis 5, **dadurch gekennzeichnet, daß** der Keil (140) mit Hilfe eines einzigen Materials hergestellt ist und sein Mittelteil (152) Ausnehmungen (154, 156) aufweist, die ihn vollständig durchqueren.
